# EUROPEAN PATENT APPLICATION

(11) **EP 2 526 908 A1**
(43) Date of publication of application: **28.11.2012**
(21) Application number: 11734676.7
(22) Date of filing: 19.01.2011
(51) Int. Cl.: A61F 5/44, A61F 13/42, A61F 13/49

(54) **EXCRETION MANAGEMENT SYSTEM, EXCRETION DETECTION DEVICE, AND RECEPTION DEVICE**

(30) Priority: 19.01.2010 JP 2010009305
(71) Applicant: Unicharm Corporation, Ehime 799-0111 (JP)
(72) Inventor: SUZUKI, Miou, Kanonji-shi Kagawa 769-1602 (JP); FUJIOKA, Yoshihisa, Kanonji-shi Kagawa 769-1602 (JP)
(74) Representative: Kensett, John Hinton
(86) International application number: PCT/JP2011/050868
(87) International publication number: WO 2011/090070

(57) **Abstract**

An excretion detection device 100 comprises a radio transmission unit 180 that is configured to transmit radio signals to a transmitter-receiver 20, the radio signals containing temperature information detected by a temperature sensor 130 and a diaper identifier which is unique to a disposable diaper. The transmitter-receiver 20 comprises: a diaper identifier authentication unit 23 configured to authenticate the diaper identifier which is contained in the radio signals received from the excretion detection device 100 when a field intensity of the received radio signals is higher than a predetermined threshold; and a reception processing unit 25 configured to execute processing of the diaper identifier authenticated by the identifier authentication unit and the temperature information that is associated with the authenticated diaper identifier.

## Description

### [Technical Field]

The present invention relates to an excretion management system, an excretion detection device, and a reception device so as to detect the excretion of a bodily waste from a living body wearing a disposable diaper.

### [Background Art]

For absorbent articles such as disposable diapers, excretion detection devices that detect the excretion of urine or stool, for example, from a wearer (living body) of an absorbent article are known. For example, a disposable diaper that is installed with an excretion detection device having a temperature sensor and a passive-type IC tag is proposed (see Patent Document 1). According to such an excretion detection device, the temperature sensor and the IC tag operate with electric power supplied by radio from the reception device installed in the proximity of a disposable diaper (for example, under the bed of a person who needs care). As a result, the information showing temperature inside the disposable diaper is transmitted to an excretion management server installed at a nurse station, for example, through the reception device.

There are also proposed methods in which electrodes configured from materials having different ionization tendencies are placed in contact with a bodily waste from a wearer so as to electrify and operate a temperature sensor and an active-type IC tag with the electromotive force (for example, Patent Documents 2 and 3). It is also possible to consider electrification with, instead of a bodily waste, an electrolyte solution that seeps out from a bag filled with the electrolyte solution in which electrolytes are dissolved in a solvent such as water at the beginning of using the disposable diaper.

That is, by applying the principle of a voltaic cell, it becomes possible to install an active-type IC tag on a disposable diaper, allowing a transmission power of radio signals to increase. As a result, it becomes unnecessary to set up a reception device, such as a passive-type IC tag, in the proximity of a disposable diaper, and the information which indicates temperature inside a disposable diaper can be transmitted to an excretion management server through a reception device which is set up at a position relatively far (at about several meters) from the disposable diaper.

### [Prior Art]

### [Patent Document]

[Patent Document 1] Japanese Patent Application Publication No. 2006-296566 (Pages 5 and 6, Fig. 1)
[Patent Document 2] Japanese Patent Application Publication No. 2002-277435 (Page 3, Fig. 4)
[Patent Document 3]: Japanese Patent Application Publication No. H4-138155 (Pages 2 and 3, Fig. 1)

### [Summary of the Invention]

However, the conventional excretion detection devices which are electrified by using a bodily waste or an electrolyte solution have the following problems. Specifically, in case of the excretion detection devices, the devices might keep transmitting radio signals even from a used disposable diaper that is removed from a wearer. In this case, it is probable that an excretion management server makes a false recognition of excretion based on the information transmitted from a used disposable diaper, which is thus unfavorable.

In order to avoid those problems, a used disposable diaper may be kept sufficiently away from a reception device, or an IC tag installed in a used disposable diaper may be bent and then fractured. Instead, a used disposable diaper may be put inside a bag having a radio wave shielding property and then disposed of. However, these measures are quite troublesome, and it is not preferable to compel users to take those measures.

Thus, it is an object of the present invention to provide, in electrifying with a bodily waste and the like, an excretion management system and an excretion detection device by which it is possible to reduce a possibility of falsely recognizing that there is an excretion, which is so caused by a radio signal transmitted from a used disposable diaper, without compelling users to take troublesome measures.

In order to solve the aforementioned problem, the present invention has a following feature. That is, the present invention is an excretion management system (excretion management system 1), comprising: an excretion detection device (excretion detection device 100) that is installed in a disposable diaper (disposable diaper 10A, 10B) and that is configured to detect an excretion of a bodily waste (urine or stool) from a living body (wearer W) wearing the disposable diaper; and a reception device (transmitter-receiver 20) configured to receive radio signals transmitted from the excretion detection device; wherein the excretion detection device comprises: a power supply unit (power supply unit 160) having electrodes (electrode 121, electrode 122) configured from materials having different ionization tendencies; a temperature sensor (temperature sensor 130) that operates on electric power generated by the power supply unit and that is configured to detect environmental information (e.g., temperature information) which changes along with an excretion from the living body; and a radio transmission unit (radio transmission unit 180) that operates on electric power generated by the power supply unit and that is configured to transmit radio signals to the reception device, the radio signals containing the environmental information detected by the temperature sensor and a diaper identifier which is unique to the disposable diaper (e.g., ID:0001); and
wherein the reception device comprises:
an identifier authentication unit (diaper identifier authentication unit 23) configured to authenticate the diaper identifier which is contained in the radio signals received from the excretion detection device, in accordance with a predetermined condition (e.g., when a field intensity of the received radio signals is higher than a predetermined threshold); and a reception processing unit (reception processing unit 25) configured to execute processing of the diaper identifier authenticated by the identifier authentication unit and the environmental information that is associated with the authenticated diaper identifier.

According to the aforementioned embodiment, preferably, the identifier authentication unit authenticates the diaper identifier when a field intensity of the radio signals is higher than a predetermined threshold

According to the aforementioned embodiment, preferably, the diaper identifier is associated with a manufacturing lot of the disposable diapers.

Another feature of the present invention is an excretion detection device that is installed in a disposable diaper and that is configured to detect an excretion of a bodily waste from a living body wearing the disposable diaper, the excretion detection device comprising: a power supply unit having electrodes configured from materials having different ionization tendencies; a temperature sensor that operates on electric power generated by the power supply unit and that is configured to detect environmental information which changes along with an excretion from the living body; and a radio transmission unit that operates on electric power generated by the power supply unit and that is configured to transmit radio signals to the reception device, the radio signals containing the environmental information detected by the temperature sensor and a diaper identifier which is unique to the disposable diaper.

Another feature of the present invention is a reception device that is installed in a disposable diaper and receives radio signals which are transmitted from an excretion detection device configured to detect an excretion of a bodily waste from a living body wearing the disposable diaper, the reception device comprising: an identifier authentication unit configured to authenticate a diaper identifier that is contained in the radio signals received from the excretion detection device and that is unique to the disposable diaper, in accordance with a predetermined condition; and a reception processing unit configured to execute processing of the diaper identifier authenticated by the identifier authentication unit and the environmental information associated with the authenticated diaper identifier.

According to the present invention, it is possible to provide an excretion management system and an excretion detection device by which it is possible to reduce a possibility of falsely recognizing that there is an excretion, which is so caused by a radio signal transmitted from a used disposable diaper without compelling users to take troublesome measures.

### [Brief Description of the Drawings]

[Fig. 1] Fig. 1 is a diagram showing an entire schematic configuration of an excretion management system 1 according to an embodiment of the present invention.
[Fig. 2] Fig. 2 is a developed plan view of a disposable diaper 10A in which an excretion detection device 100 is embedded, and a schematic illustration of the appearance of the excretion detection device 100 according to the embodiment of the present invention.
[Fig. 3] Fig. 3 is a block configuration diagram showing the functions of the excretion detection device 100 and a transmitter-receiver 20 according to the embodiment of the present invention.
[Fig. 4] Fig. 4 shows the excretion management flow using the excretion management system 1 according to the embodiment of the present invention.

### [Description of Embodiments]

Subsequently, an embodiment of an excretion detection device and an absorbent article according to the present invention is explained by referring to the figures. In the following description of the drawings, the same or similar reference numerals are used to designate the same or similar portions. It is appreciated that the drawings are schematically shown and the ratio and the like of each dimension are different from the real ones.

Accordingly, specific dimensions and the like should be determined in consideration of the explanation below. Moreover, among the drawings, the respective dimensional relations or ratios may differ.

### (1) Entire schematic configuration of excretion management system

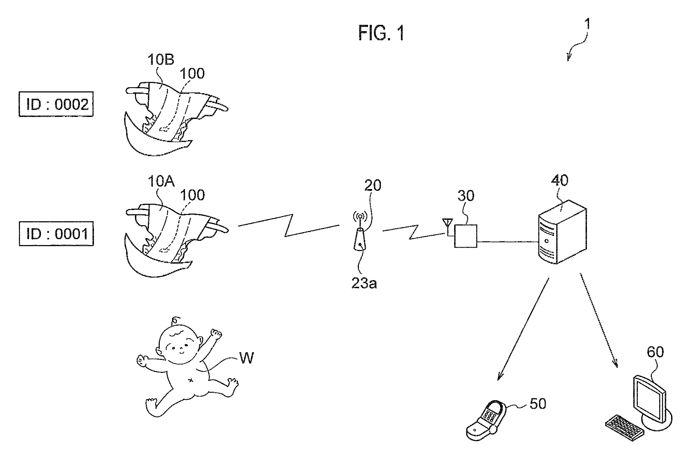

Fig. 1 is a diagram showing an entire schematic configuration of an excretion management system 1 according to the present embodiment. As illustrated in Fig. 1, the excretion management system 1 is configured by a transmitter-receiver 20, an excretion management server 40, and an excretion detection device 100.

A disposable diaper 10A and a disposable diaper 10B are absorbent articles which are worn by a wearer W (living body) and absorb a bodily waste, such as urine or stool from the wearer W. The disposable diaper 10A and the disposable diaper 10B are installed with the excretion detection device 100. Although the disposable diaper 10A and the disposable diaper 10B are open-type (tape-type), they may be pant-type disposable diapers.

The excretion detection device 100 detects that there is an excretion of a bodily waste from the wearer W. The excretion detection device 100 is configured by an active-type IC tag that can transmit radio signals to the transmitter-receiver 20, a temperature sensor, and the like, and repeatedly transmits radio signals containing temperature information (environmental information) inside a disposable diaper at a predetermined frequency. The detailed configuration of the excretion detection device 100 is described later.

The excretion detection device 100 is provided with a diaper identifier that is characteristic to each excretion detection device 100, in other words, characteristic to each disposable diaper. Accordingly, each disposable diaper can be uniquely specified. In the present embodiment, "ID: 0001" is assigned to the disposable diaper 10A and "ID: 0002" is assigned to the disposable diaper 10B.

The transmitter-receiver 20 receives the radio signals transmitted from the excretion detection device 100, and converts the received radio signals into radio signals according to a predetermined communication system. The transmitter-receiver 20 also identifies a diaper identifier contained in the radio signals. In the present embodiment, the transmitter-receiver 20 converts the radio signals received from the excretion detection device 100 into radio signals for the Personal Handy Phone (PHS) System, and transmits the radio signals to a radio base station 30.

The transmitter-receiver 20 is a rectangular parallelepiped of several centimeters or less on each side, and can be mounted on a bed or the wearer W in the form of a name tag. Moreover, in order to keep radio communication between the transmitter-receiver 20 and the excretion detection device 100, it is necessary to position the transmitter-receiver 20 and the excretion detection device 100 within a fixed distance (within several meters). Additionally, the detailed configuration of the transmitter-receiver 20 will be described below.

The radio base station 30 receives radio signals from the transmitter-receiver 20. In the present embodiment, the radio base station 30 transmits and receives radio signals in accordance with the PHS system. The radio base station 30 is connected to the excretion management server 40 via a wire communication network (not shown in the figure).

The excretion management server 40 stores a plurality of change patterns of the environmental information (such as the temperature, humidity, and odor) concerning defecation and urination. The excretion management server 40 determines whether or not there has been urination or defecation from the wearer W based on temperature information repeatedly transmitted from the excretion detection device 100 through the transmitter-receiver 20 and the radio base station 30. Upon determining the occurrence of urination or defecation, the excretion management server 40 transmits an email indicating the same to a pre-registered cellular phone terminal 50 or the personal computer 60. As the excretion management server 40 sends such an email to the address that is already registered at the cellular phone terminal 50 or the personal 50 or the personal computer 60, a guardian (caregiver) of the wearer W who confirmed the email can promptly take proper action such as the changing of a disposable diaper.

### (2) Layout position and structure of excretion detection device

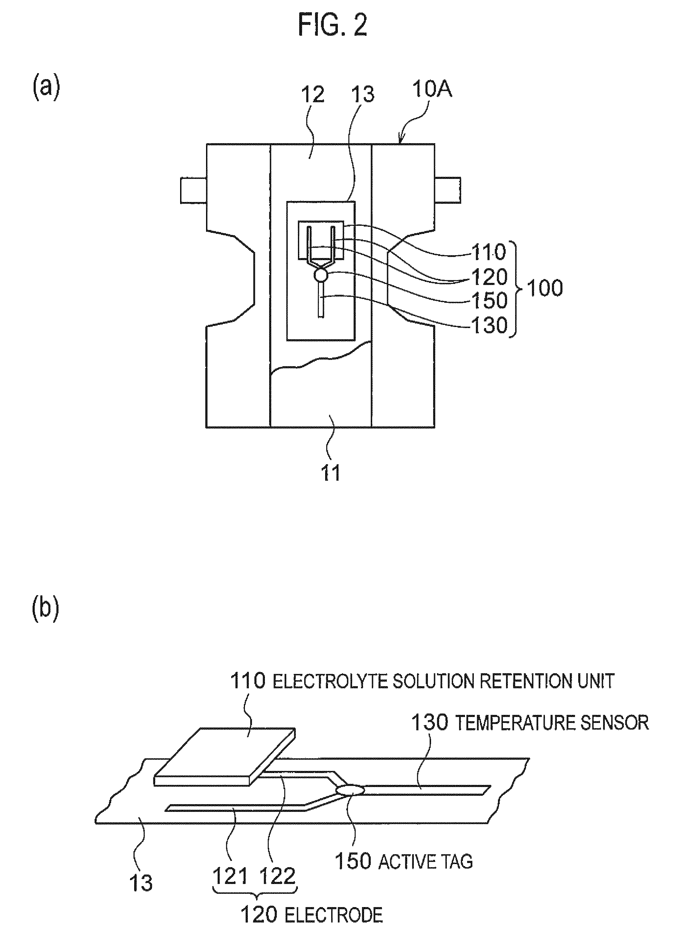

Fig. 2 (a) is a developed plan view of the disposable diaper 10A in which the excretion detection device 100 is embedded. Fig. 2 (b) is a schematic illustration of the appearance of the excretion detection device 100.

As illustrated in Fig. 2 (a), the disposable diaper 10A (disposable diaper 10B) has a topsheet 11, a backsheet 12, and an absorber 13. The topsheet 11 contacts the skin of the wearer W (living body). The topsheet 11 is configured from a material that allows liquids such as urine to pass through. The backsheet 12 contacts the clothes of the wearer W. The backsheet 12 is configured from a material that does not allow liquids to pass through. The absorber 13 absorbs a bodily waste, such as urine or stool. The absorber 13 is configured from hydrophilic fibers such as ground pulp. The disposable diaper 10A can be manufactured according to a well-known method (for example, Japanese Patent Application Publication No. JP2003-339771).

The excretion detection device 100 is provided between the topsheet 11 and the absorber 13. The excretion detection device 100 may be provided between the backsheet 12 and the absorber 13, however, in order to be sure that the stool, which has a higher viscosity as compared to urine, is brought in contact with an electrode unit 120 of the excretion detection device 100, it is desired that the excretion detection device 100 be provided between the topsheet 11 and the absorber 13.

The excretion detection device 100 has an electrolyte solution retention unit 110, the electrode unit 120, a temperature sensor 130, and an active tag 150.

The electrode unit 120 is arranged below the electrolyte solution retention unit 110 (towards the absorber 13). In order to prevent the temperature sensor 130 from contacting the electrolyte solution seeping out from the electrolyte solution retention unit 110, the sensor is arranged on the opposite side, with the active tag 150 placed as a reference, from an electrode 121 and an electrode 122. The electrode 121 and the electrode 122 are arranged at a position capable of contacting the bodily waste from the wearer W and the electrolyte solution seeping out from the electrolyte solution retention unit 110. That is, the electrode 121 and the electrode 122 can be contacted to the electrolyte solution seeping out from the electrolyte solution retention unit 110 before the excretion of urine and the like.

The active tag 150 is connected to the electrode unit 120 and the temperature sensor 130, and is arranged at a position not to overlap the electrolyte solution retention unit 110.

### (3) Block configuration showing the functions of excretion detection device 100

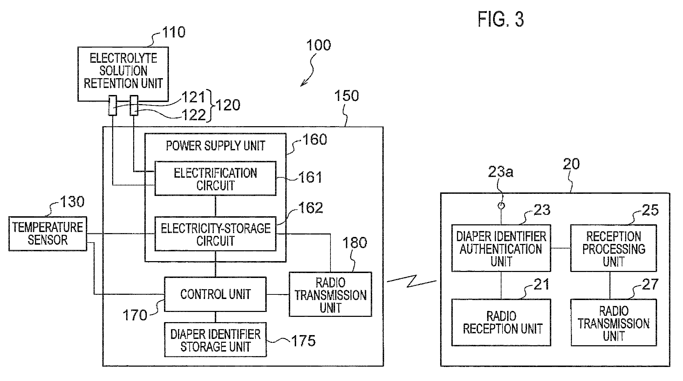

Fig. 3 is a block configuration diagram showing the functions of the excretion detection device 100 and the transmitter-receiver 20. As illustrated in Fig. 3, the excretion detection device 100 has the electrolyte solution retention unit 110, the electrode unit 120, the temperature sensor 130, and the active tag 150. The active tag 150 is configured by a power supply unit 160, a control unit 170, a diaper identifier storage unit 175, and a radio transmission unit 180.

The electrolyte solution retention unit 110 holds an electrolyte solution in which electrolytes are dissolved in a solvent such as water. For the electrolyte solution retention unit 110, an aqueous solution containing sodium chloride (NaCl) at about 1 wt% to 5 wt%, for example, can be used. That is, an NaCl aqueous solution similar to the urinary constituent is used as the electrolyte solution. The electrolyte solution retention unit 110 is a bag-shaped container which can store a liquid. In the present embodiment, the electrolyte solution retention unit 110 is configured from a thermoplastic polyolefin film.

The electrolyte solution retention unit 110 makes an electrolyte solution seep out therefrom by a predetermined action to the electrolyte solution retention unit 110, for example, by pressing the electrolyte solution retention unit 110 from the topsheet 11 side. Specifically, by pressing the electrolyte solution retention unit 110, the bag-shaped electrolyte solution retention unit 110 configured from a thermoplastic film is torn, and the electrolyte solution seeps out to the periphery of the electrode unit 120. As a result, the electrolyte solution comes in contact with the electrode 121 and the electrode 122.

Moreover, the electrolyte solution retention unit 110 may hold only a solvent such as water, and sodium chloride powder may be arranged near the electrolyte solution retention unit 110. In this case, the sodium chloride powder dissolves in the water that seeps out from the electrolyte solution retention unit 110, and an electrolyte solution is then made.

The electrode unit 120 is arranged below the electrolyte solution retention unit 110 (towards the absorber 13) and above the absorber 13. A pair of electrode 121 and electrode 122 configuring the electrode unit 120 are connected to the power supply unit 160. The electrode 121 and the electrode 122 are configured to use materials having different ionization tendencies. In the present embodiment, a combination of aluminum and silver is used as the electrode 121 and the electrode 122. That is, the power supply unit 160 having such an electrode unit 120 functions as a so-called voltaic cell through the interposition of the electrolyte solution or the bodily waste between the electrode 121 and the electrode 122. A voltaic cell is a cell that generates electric current by moving electrons through the immersion of electrodes using a metal with a large ionization tendency and a metal with a small ionization tendency, in an electrolyte solution.

As described above, the electrode 121 and the electrode 122 can be contacted to the electrolyte solution seeping out from the electrolyte solution retention unit 110 before the excretion of urine and the like. Therefore, a difference in potential occurs between the electrode 121 and the electrode 122 due to the electrolyte solution prior to excretion, and due to the electrolyte solution or the bodily waste after excretion, and electromotive force is obtained.

The temperature sensor 130 detects the environmental information which changes due to the excretion from the wearer W. Specifically, in the disposable diaper 10A, the temperature sensor 130 detects the temperature of the region where the bodily waste is excreted (the region in which the absorber 13 is provided) as the environmental information. The temperature sensor 130 is operated by the electric power generated by the power supply unit 160.

In the present embodiment, a thin-film thermistor (hereinafter, thermistor) is used as the temperature sensor 130. In the temperature sensor 130, a synthetic-resin film on which the electrode for detecting the urine or stool, and the electrode for supplying electric power to the thermistor are marked with a conductive ink is used. A thermistor that is easily affected by the surrounding temperature at which the heat capacity is less, for example, the ET-103 thermistor manufactured by SEMITEC CORPORATION, is desired to be used as the temperature sensor 130.

The active tag 150 is an active-type IC tag with which the electrode unit 120 and the temperature sensor 130 are connected. That is, the active tag 150 can transmit a radio signal without depending on another radio communication device, for example.

The power supply unit 160 has an electrification circuit 161 and an electricity-storage circuit 162. The electrode 121 and the electrode 122 are connected to the electrification circuit 161. The electrification circuit 161 is electrified due to the difference in potential that occurs between the electrode 121 and the electrode 122, and stores the generated electric power in the electricity-storage circuit 162. The electricity-storage circuit 162 is configured from a capacitor, for example.

The control unit 170 is operated by the electric power generated by the power supply unit 160. Specifically, based on the value output from the temperature sensor 130, the control unit 170 generates data indicating temperature information, and then outputs the generated data to the radio transmission unit 180.

The diaper identifier storage unit 175 memorizes a diaper identifier (for example, ID: 0001) that is unique to the excretion detection device 100, more specifically, that is unique to the disposable diaper 10A (or the disposable diaper 10B) in which the excretion detection device 100 is installed. In the present embodiment, a diaper identifier corresponds to a manufacturing lot of disposable diapers. The first two digits of ID: 0001, for example, correspond to the manufacturing lot. Furthermore, the number of digits of a diaper identifier, for example, is not limited to the one in the present embodiment, and may be appropriately changed depending on the amount of necessary information.

The radio transmission unit 180 is operated by the electric power generated by the power supply unit 160. The radio transmission unit 180 transmits temperature information detected by the temperature sensor 130 and radio signals containing a diaper identifier (for example, ID: 0001) to the transmitter-receiver 20. In the present embodiment, the radio transmission unit 180 repetitively transmits the radio signals at a predetermined frequency (for example, 10 seconds).

As described above, because the excretion detection device 100 functions as a voltaic cell before and after excretion, the excretion detection device 100 is a battery-less type device in which a battery is not installed for operating the temperature sensor 130, the control unit 170, and the radio transmission unit 180.

### (4) Block configuration showing the functions of transmitter-receiver 20

The transmitter-receiver 20 has a radio reception unit 21, a diaper identifier authentication unit 23, a reception processing unit 25, and a radio transmission unit 27.

The radio reception unit 21 receives radio signals transmitted from the excretion detection device 100, and then outputs the temperature information contained in the received radio signals to the reception processing unit 25. Moreover, the radio reception unit 21 outputs the diaper identifier contained in the radio signals to the diaper identifier authentication unit 23.

The diaper identifier authentication unit 23 authenticates the diaper identifier contained in the radio signals that are received from the excretion detection device 100, in accordance with a predetermined condition. Specifically, the diaper identifier authentication unit 23 authenticates a diaper identifier contained in radio signals when the field intensity of the received radio signals is higher than a predetermined threshold.

When the diaper identifier authentication unit 23 authenticates a new diaper identifier, a LED display unit 23a turns on for a fixed period (for example, ten seconds) with a color different from the color during the operation period (at the state of periodically receiving radio signals). The LED display unit 23a is provided at the surface of the transmitter-receiver 20 (see Fig. 1). Moreover, the display of the LED display unit 23a in case of authenticating a new diaper identifier is not limited to the lightening of a color different from the color during the operation period but may be in other forms such as blinking light over a fixed period.

The reception processing unit 25 executes the processing of a diaper identifier and temperature information. Specifically, the reception processing unit 25 executes the processing (configuration of a data frame, for example) that is necessary so as to transmit an authenticated diaper identifier and temperature information which corresponds to the diaper identifier from the radio transmission unit 27.

More specifically, the reception processing unit 25 executes the processing of temperature information contained in the same radio signals as the diaper identifier that is authenticated by the diaper identifier authentication unit 23. In other words, when the diaper identifier authentication unit 23 authenticates a new diaper identifier (for example, ID: 0002), the reception processing unit 25 ignores radio signals containing a diaper identifier (such as ID: 0001) that is different from the authenticated diaper identifier after the authentication, and does not relay temperature information that is contained in the radio signals to the excretion management server 40.

Instead of such processing, the reception processing unit 25 may notify the excretion management server 40 that a new diaper identifier (for example, ID: 0002) is authenticated by the diaper identifier authentication unit 23. In this case, although it is possible to keep transmitting temperature information that corresponds to an old diaper identifier different from a diaper identifier which is newly authenticated by the diaper identifier authentication unit 23 to the excretion management server 40, the excretion management server 40 may execute the processing which targets only temperature information that corresponds to the newly authenticated diaper identifier.

The radio transmission unit 27 transmits radio signals based on the PHS system to the radio base station 30. Specifically, the radio transmission unit 27 transmits radio signals containing a diaper identifier and temperature information output from the reception processing unit 25.

### (5) Excretion management method using excretion management system

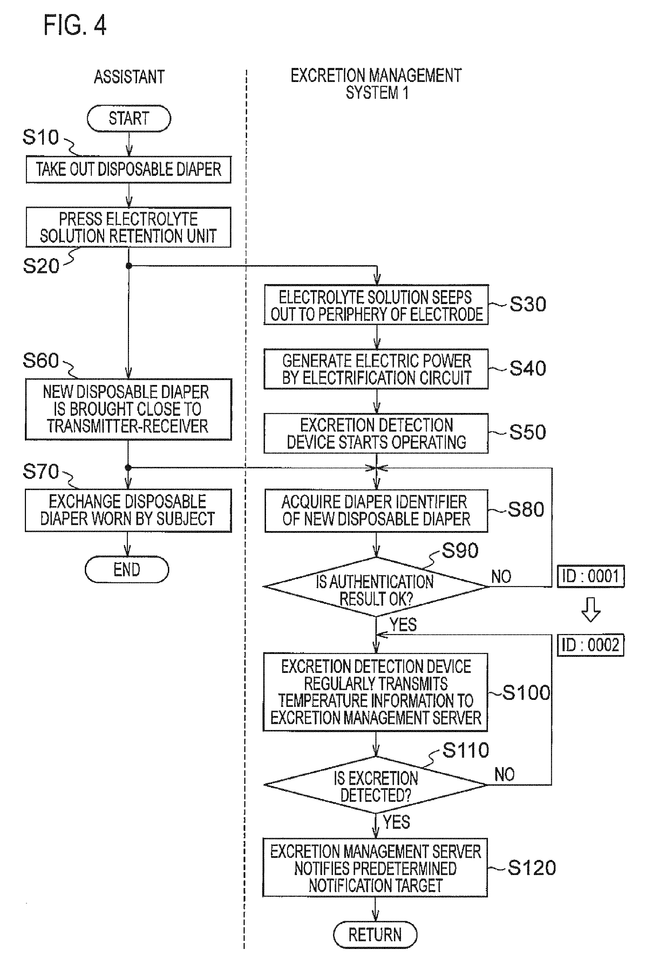

Fig. 4 shows the excretion management flow using the aforementioned excretion management system 1. Specifically, Fig. 4 is a flow diagram from the step in which the urination or defecation from the wearer W wearing the disposable diaper 10A is detected by the excretion detection device 100 until the step in which the disposable diaper 10A is exchanged with the disposable diaper 10B. It is assumed that the disposable diaper 10A (ID: 0001) has been already worn by the wearer W herein.

As shown in Fig. 4, in step S10, the guardian or caregiver (hereinafter, assistant) who assists an object person such as a person who needs care to put on a disposable diaper, takes out the unused disposable diaper 10B (ID: 0002) from a package and the like.

In step S20, the assistant presses a portion of the electrolyte solution retention unit 110 that is embedded under the topsheet 11 of the disposable diaper 10B. Due to this action by the assistant, the bag-shaped electrolyte solution retention unit 110 is torn.

In step S30, an electrolyte solution (sodium chloride solution) seeps out from the electrolyte solution retention unit 110 to the periphery of the electrode unit 120.

In step S40, the power supply unit 160 (electrification circuit 161) is electrified due to the interposition of the electrolyte solution between the electrode 121 and the electrode 122, and generates electric power.

In step S50, the excretion detection device 100 starts operating as a result of the electric power generated by the power supply unit 160.

In step S60, the assistant moves the new disposable diaper 10B close to the transmitter-receiver 20.

In step S70, the assistant puts the disposable diaper 10B on an object person such as an infant and a toddler. Accordingly, the assistant completes the measure of putting on the disposable diaper 10B.

In step S80, the transmitter-receiver 20 obtains a diaper identifier of the new disposable diaper 10B.

In step S90, the transmitter-receiver 20 determines whether or not it would authenticate the obtained diaper identifier. Specifically, when a field intensity of radio signals containing a diaper identifier is higher than a predetermined threshold, the transmitter-receiver 20 authenticates the diaper identifier contained in the radio signals. In other words, since the assistant has been moved the new disposable diaper 10B close to the transmitter-receiver 20 in the measure in step S60, the transmitter-receiver 20 can receive radio signals at a field intensity which is higher than that in a normal state (in which the disposable diaper is placed apart from the transmitter-receiver 20 (at about several meters)). As a result, the transmitter-receiver 20 can determine that the diaper will be exchanged with the new disposable diaper 10B.

Moreover, the transmitter-receiver 20 updates the ID: 0001 that has been stored at the diaper identifier storage unit 175, to ID: 0002, and from this point onward, transmits only the temperature information that corresponds to the ID: 0002 to the excretion management server 40 without transmitting the temperature information that corresponds to the ID: 0001.

In step S100, the radio transmission unit 180 periodically transmits the temperature information that is obtained by the temperature sensor 130, to the excretion management server 40. Specifically, the radio transmission unit 180 transmits the temperature information to the excretion management server 40 through the transmitter-receiver 20 and the radio base station 30 as described above.

In step S110, the excretion management server 40 determines whether or not the pattern of temperature changes matches the originally stored pattern based on the received temperature information. In the present embodiment, the excretion management server 40 stores the temperature change pattern that occurs as a result of urination, and the temperature change pattern that occurs as a result of defecation.

For example, when the temperature change patterns are compared between the time of defecation and the time of urination, the temperature rises more sharply and the convergence of temperature changes is faster during urination than defecation. On the other hand, the rise in the temperature is slower during defecation, and the convergence of temperature change is also slower. Based on such differences in the temperature change pattern, the excretion management server 40 identifies urination and defecation.

When the pattern of temperature changes based on the received temperature information matches the pattern of temperature changes due to urination or defecation (YES in step S110), the excretion management server 40 sends the email which indicates there has been urination or defecation to the address that has been already registered (the cellular phone terminal 50 or the personal computer 60) in step S120.

According to the excretion management system 1 explained above, the diaper identifier contained in radio signals received from the excretion detection device 100 is authenticated in accordance with a predetermined condition. Specifically, the field intensity of radio signals that the transmitter-receiver 20 receives becomes higher than a predetermined threshold as an assistant who assists to put on a disposable diaper brings a new disposable diaper close to the transmitter-receiver 20, and the diaper identifier of the new disposable diaper is then authenticated. Furthermore, at the transmitter-receiver 20, the processing of the authenticated diaper identifier and the temperature information that corresponds to the diaper identifier is executed.

Therefore, it is possible to accurately reduce a possibility of falsely recognizing that there is an excretion from a radio signal transmitted from a used disposable diaper, with only an extremely simple process of bringing a new disposable diaper close to the transmitter-receiver 20 by an assistant.

Since disposable diapers are individually authenticated by a diaper identifier, actual conditions such as the time-of-use and the excretion timing of each disposable diaper can be individually understood, and the information which contributes to product development can be obtained.

In the present embodiment, a diaper identifier is associated with the manufacturing lot of disposable diapers. Accordingly, precision such as the traceability of disposable diapers can improve.

In the present embodiment, the electrode unit 120 is arranged at a position capable of contacting the bodily waste from the wearer W and the electrolyte solution that is held by the electrolyte solution retention unit 110. Furthermore, the electrode unit 120 can be contacted to the electrolyte solution even before the excretion of the wearer W. Thus, it becomes possible to electrify with the power supply unit 160 even before urination or defecation.

### (6) Other embodiments

As described above, the present invention is disclosed through the above embodiments. However, it should not be interpreted that the statements and drawings constituting a part of the present disclosure limit the present invention. From this disclosure, a variety of alternate embodiments, examples, and applicable techniques become apparent to one skilled in the art.

For example, in the above-noted embodiment, the transmitter-receiver 20 authenticates a diaper identifier contained in radio signals. However, when a diaper identifier cannot be obtained even by bringing a new disposable diaper close to the transmitter-receiver 20 due to poor transmission status, for example, an assistant may directly input a diaper identifier to the transmitter-receiver 20. In this case, it is acceptable if a diaper identifier is displayed at the proper position of a disposable diaper and the transmitter-receiver 20 has an input unit (such as ten key pad) of a diaper identifier.

In the aforementioned embodiment, the transmitter-receiver 20 relays temperature information to the excretion management server 40, but the transmitter-receiver 20 may manage temperature information without relaying to the excretion management server 40.

In the embodiment described above, although temperature information is transmitted by using the radio transmission unit 180, an alarm such as a buzzer, instead of the transmission of temperature information by the radio transmission unit 180, may be activated.

In the aforementioned embodiment, the electrolyte solution retention unit 110 is torn by pressing the electrolyte solution retention unit 110 from the topsheet 11 side, and the electrolyte solution seeps out from the electrolyte solution retention unit 110. However, the electrolyte solution may seep out by a process other than pressing. For example, the electrolyte solution retention unit 110 may be torn by pulling a thread-like member connected to the electrolyte solution retention unit 110.

Furthermore, the excretion detection device 100 is not just installed in a diaper, but may also be installed in an absorbent article such as a sanitary napkin. Additionally, the absorbent article is not just used for human beings, but may also be used for animals, such as pets.

As described above, it is a matter of course that the present invention includes various embodiments not described here. Therefore, the technical range of the present invention is to be defined only by the inventive specific matter according to the adequate claims from the above description.

The entire contents of Japanese Patent Application No. 2010-009305 (filed on January 19, 2010) are incorporated in the present specification by reference.

### [Industrial Applicability]

According to the present invention, it is possible to provide the excretion management system and the excretion detection device by which it is possible to reduce a possibility of falsely recognizing that there is an excretion, by a radio signal transmitted from a used disposable diaper without making a user take a troublesome measure.

### [Explanation of the Reference Signs]

1: excretion management system, 10A, 10B: disposable diaper, 11: topsheet, 12: backsheet, 13: absorber, 20: transmitter-receiver, 21: radio reception unit, 23: diaper identifier authentication unit, 23a: LED display unit, 25: reception processing unit, 27: radio transmission unit, 30: radio base station, 40: excretion management server, 50: cellular phone terminal, 60: personal computer, 100: excretion detection device, 110: electrolyte solution retention unit, 120: electrode unit, 121, 122: electrode, 130: temperature sensor, 150: active tag, 160: power supply unit, 161: electrification circuit, 162: electricity-storage circuit, 170: control unit, 175: diaper identifier storage unit, 180: radio transmission unit, W: wearer

## Claims

1. An excretion management system, comprising:
an excretion detection device that is installed in a disposable diaper and that is configured to detect an excretion of a bodily waste from a living body wearing the disposable diaper; and
a reception device configured to receive radio signals transmitted from the excretion detection device;
wherein
the excretion detection device comprises:
a power supply unit having electrodes configured from materials having different ionization tendencies;
a temperature sensor that operates on electric power generated by the power supply unit and that is configured to detect environmental information which changes along with an excretion from the living body; and
a radio transmission unit that operates on electric power generated by the power supply unit and that is configured to transmit radio signals to the reception device, the radio signals containing the environmental information detected by the temperature sensor and a diaper identifier which is unique to the disposable diaper; and
wherein the reception device comprises:
an identifier authentication unit configured to authenticate the diaper identifier which is contained in the radio signals received from the excretion detection device, in accordance with a predetermined condition; and
a reception processing unit configured to execute processing of the diaper identifier authenticated by the identifier authentication unit and the environmental information that is
associated with the authenticated diaper identifier.

2. The excretion management system according to claim 1, wherein the identifier authentication unit authenticates the diaper identifier when a field intensity of the radio signals is higher than a predetermined threshold.

3. The excretion management system according to claim 1 or 2, wherein the diaper identifier is associated with a manufacturing lot of the disposable diapers.

4. An excretion detection device that is installed in a disposable diaper and that is configured to detect an excretion of a bodily waste from a living body wearing the disposable diaper, the excretion detection device comprising:
a power supply unit having electrodes configured from materials having different ionization tendencies;
a temperature sensor that operates on electric power generated by the power supply unit and that is configured to detect environmental information which changes along with an excretion from the living body;
and
a radio transmission unit that operates on electric power generated by the power supply unit and that is configured to transmit radio signals to the reception device, the radio signals containing the environmental information detected by the temperature sensor and a diaper identifier which is unique to the disposable diaper.

5. A reception device that is installed in a disposable diaper and receives radio signals which are transmitted from an excretion detection device configured to detect an excretion of a bodily waste from a living body wearing the disposable diaper, the reception device comprising:
an identifier authentication unit configured to authenticate a diaper identifier that is contained in the radio signals received from the excretion detection device and that is unique to the disposable diaper, in accordance with a predetermined condition; and
a reception processing unit configured to execute processing of the diaper identifier authenticated by the identifier authentication unit and the environmental information associated with the authenticated diaper identifier.
